# EUROPEAN PATENT APPLICATION

(11) **EP 2 213 250 A1**
(43) Date of publication of application: **04.08.2010**
(21) Application number: 10250140.0
(22) Date of filing: 28.01.2010
(51) Int. Cl.: A61B 17/06, A61B 17/00, A61B 17/02, A61B 17/34

(54) **Suture management system for surgical portal apparatus including internal tubes**

(30) Priority: 30.01.2009 US 148483 P; 14.12.2009 US 636809
(71) Applicant: Tyco Healthcare Group LP, North Haven, CT 06473 (US)
(72) Inventor: Hathaway, Peter, Lebanon, CT 06249 (US)
(74) Representative: Soames, Candida Jane

(57) **Abstract**

A surgical portal apparatus for use in surgical procedures incorporating at least one suture includes a portal housing defining a longitudinal axis and having a longitudinal opening therethrough for receiving a surgical object and a sleeve extending from the portal housing and including one or more channels formed along the length of the sleeve. The channels are configured to receive the at least one suture.

## Description

### CROSS REFERENCE TO RELATED APPLICATION

The present application claims the benefit of and priority to U.S. Provisional Application Serial No. 61/148,483 filed on January 30, 2009, the entire contents of which are incorporated herein by reference.

### BACKGROUND

### Technical Field

The present disclosure relates to trocars and other surgical portal apparatus, and more particularly, relates to surgical portal apparatus including a suture management system that includes internal tubes.

### Background of Related Art

Trocars and other surgical portal apparatus are known, as are myriad procedures that may be preformed using such assemblies. Many of the minimally invasive procedures performed through access assemblies necessitate or are simplified by the use of one or more sutures passing through the surgical portal apparatus. Sutures extending into a body cavity through a surgical portal apparatus may be used to, for example, temporarily retain tissue, manipulate tissue, anchor tissue or operate peripheral devices. In an attempt to reduce the number of incision sites required to complete a given surgical procedure, a single surgical portal apparatus may be used to pass one or more sutures into a body cavity, in addition to providing access for one or more devices. A single anchor device may have numerous suture ends that extend therefrom and through the surgical portal apparatus. The sutures extending through the surgical portal apparatus may become tangled as each is manipulated or as one or more instruments are inserted and withdrawn from the assembly. Also, a surgeon may confuse the suture ends during the course of a surgery. Tangling or confusion of the suture ends may unnecessarily complicate the procedure and increase time necessary to complete the procedure.

Therefore, it would be beneficial to have a surgical portal apparatus that includes a system for managing sutures during a surgical procedure such as a laparoscopic or orthopedic procedure.

### SUMMARY

A surgical portal apparatus for use in surgical procedures incorporating at least one suture includes a portal housing defining a longitudinal axis and having a longitudinal opening therethrough for receiving a surgical object and a sleeve extending from the portal housing. One or more channels are defined along the length of the sleeve. The channels are configured to receive the at least one suture to maintain the suture in a defined relation with respect to the portal housing. The one or more channels may include a slot for receiving the at least one suture, may include open proximal and distal ends or may be integrally formed with the sleeve.

In another embodiment, a suture retaining insert for use in a surgical portal apparatus includes a sleeve configured to be selectively received in a longitudinal opening of a surgical portal apparatus and defining a longitudinal passage. The sleeve may include one or more channels integrally formed along the length of the sleeve and configured to receive at least one suture. The one or more channels may be disposed along an exterior of the sleeve or adjacent the longitudinal passage.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the einbodiment(s) given below, serve to explain the principles of the disclosure, wherein:
**FIG. 1** is a perspective side view of an surgical portal apparatus according to an embodiment of the present disclosure;
**FIG. 2** is a top view of the surgical portal apparatus of **FIG. 1****;**
**FIG. 3** is a perspective side view of a surgical portal apparatus according to another embodiment of the present disclosure;
**FIG. 4** is a top view of the surgical portal apparatus of FIG. 3;
**FIG. 5** is a perspective side view of suture management system for use in a surgical portal apparatus, according to an embodiment of the present disclosure; and
**FIG. 6** is a perspective side view of a suture management system for use in a surgical portal apparatus, according to another embodiment of the present disclosure.

### DETAILED DESCRIPTION

The surgical portal apparatus herein disclosed may be configured for use in various surgical procedures, including laparoscopic, endoscopic, arthroscopic and orthopedic surgery. The access assembly provides passage between a subject's body cavity and the outside atmosphere and is capable of receiving surgical instruments of various sizes and configurations. An embodiment of the presently disclosed access assembly is configured to receive, for example, clip appliers, graspers, dissectors, retractors, staplers, laser probes, photographic devices, endoscopes and laparoscopes, tubes, and the like. Such instruments are collectively referred to herein as "instruments" or "instrumentation."

In addition to the instruments, the access assembly also allows the passage of one or more sutures therethrough, e.g., during an arthroscopic procedure or laparoscopic procedure. When several sutures arc introduced into the subject's body through the access assembly, the sutures might tangle with each other or a surgeon may confuse the sutures during a surgical procedure. Suture tangle and/or confusion may, at the very least, inconvenience the clinicians conducting the surgical procedure. To minimize the possibility of sutures tangling with one another, the access assembly incorporates suture retaining members for holding the sutures in place.

Referring now to the drawings wherein like reference numerals illustrate similar components throughout the several views, there is illustrated surgical portal apparatus **100** in accordance with the principles of the present disclosure. As shown in the drawings and as described throughout the following description, as is traditional when referring to relative positioning on an object, the term "proximal" refers to the end of the apparatus which is closer to the user and the term "distal" refers to the end of the apparatus which is further from the user.

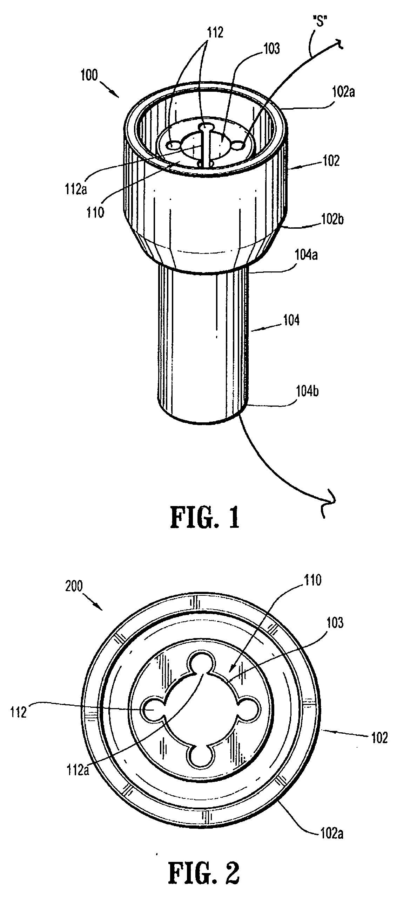

Referring initially to **FIGS. 1** and **2****,** an embodiment of a surgical portal apparatus of the present disclosure is shown generally as surgical portal apparatus **100.** Surgical portal apparatus **100** includes a portal housing **102** and a sleeve **104** extending distally from portal housing **102.** Surgical portal apparatus **100** may be configured for use with any known instrument.

Portal housing **102** defines a substantially cylindrical member having an open proximal end **102a** and a substantially open distal end **102b.** Distal end **102b** of portal housing **102** may be integrally formed with sleeve **104.** Alternatively, portal housing **102** may be configured for selectable engagement with sleeve **104.** Portal housing **102** may be constructed of plastic, polymer or other like material. Portal housing **102** may be disposable, or in the alternative, reusable. Portal housing **102** may be rigid, or alternatively, substantially flexible. Portal housing **102** may include one or more seal members (not shown) in any arrangement for receiving a surgical object in a sealing manner. Portal housing **102** may further include one or more anchors (not shown) or other suture securing means for securing one or more suture "S" extending through surgical portal apparatus **100.** Portal member **102** may additionally include an insufflation valve or port (not shown) configured to fill the body cavity of a patient with insufflation gas, saline or other suitable fluid.

Sleeve **104** is configured to be inserted through the skin into a body cavity with the aid of an obturator (not shown), or may instead, include a blade or piercing tip for penetrating through the skin and into a body cavity. Sleeve **104** forms a substantially tubular member having proximal and distal ends **104a, 104b** and defining a first longitudinal passage **103** extending therebetween. Sleeve **104** may be composed of plastic, metal, polymers or the like. Sleeve **104** may be disposable, or in the alternative, reusable. Sleeve **104** may be rigid, or alternatively, sleeve **104** may be flexible. Sleeve **104** may be open, or instead, may be configured to include one or more seal members (not shown) of any arrangement along the length thereof

Sleeve **104** further includes a suture management system **110.** Suture management system **110** includes a series of longitudinal channels **112** extending the length of sleeve **104.** Suture management system 110 may include one or more channels **112.** As shown, channels **112** include a circular cross-sectional profile; however, other configurations are envisioned. Each of channels **112** includes a slot **112a** extending at least partially along the length of sleeve **104** for selectively receiving a suture "**S**" within channel **112.** Each of channels **112** may be configured to receive one or more sutures "**S**". It is further envisioned that one or more of channels **112** may be sized and configured to receive additional instrumentation (not shown).

Still referring to **FIGS. 1** and **2**, in operation, surgical portal apparatus **100** functions similar to conventional access assemblies. As discussed above, sleeve **104** may be inserted into a body cavity through an incision with the aid of an obturator (not shown), or alternatively, sleeve **104** may be fitted with a blade to create the incision. Once received within a body cavity, surgical portal apparatus **100** may receive one or more sutures "**S**" and/or one or more devices (not shown). Initially, an endoscopic instrument (not shown) may be used to feed one or more suture **"S"** through passage **103** of sleeve **102.** Once anchored with the body cavity, suture "S" may be received in one of channels **112** through respective slot **112a.** Suture "S" may be received in channel **112** as the endoscopic instrument is withdrawn from surgical portal apparatus **100,** or alternatively, suture "S" may be placed in channel **112** by a surgeon following removal of the endoscopic device (not shown) from surgical portal apparatus **100.** It is conceivable that suture "S" may be received in channel **112** through slot **112a** while an endoscopic instrument remains in passage **103.**

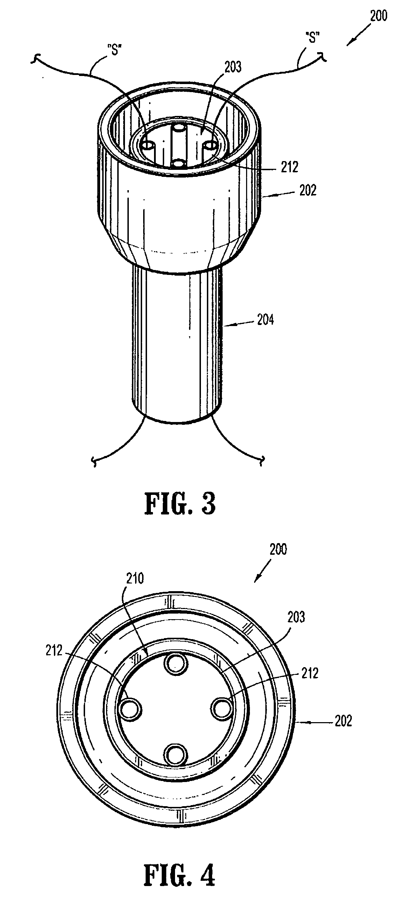

Turning now to **FIGS. 3** and **4****,** an alternate embodiment of a surgical portal apparatus of the present disclosure is shown generally as surgical portal apparatus **200.** Surgical portal apparatus **200** is substantially similar to surgical portal apparatus **100** and will only be described as relates to the differences therebetween. Surgical portal apparatus **200** includes a portal housing **202** and a sleeve **204.** Sleeve **204** includes proximal and distal end **204a, 204b** and defines a passage **203** therebetween. Sleeve **204** further includes a suture management system **200.**

Suture management system **200** includes one or more tubes **212** extending the length of sleeve **204.** Tubes **212** may be configured and sized to receive one or more sutures **"S".** Tubes **212** are positioned around an inner perimeter of sleeve **204.** Tubes **212** may be integrally formed with sleeve **204.** Alternatively, tubes **212** may be securely attached or selectively secured to sleeve **204.**

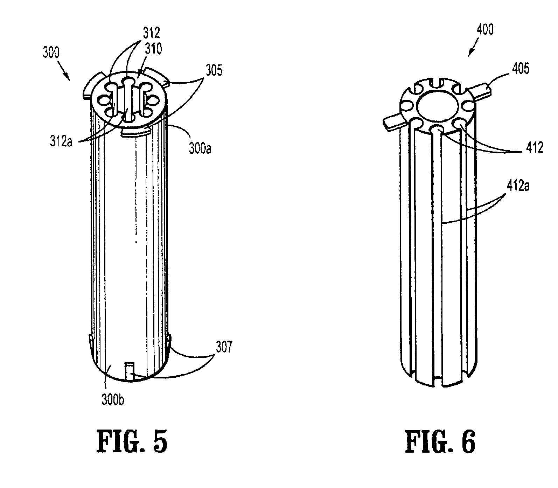

With reference now to **FIG. 5****,** another embodiment of the present disclosure is shown generally as sleeve insert **300.** Sleeve insert **300** is sized and configured to be received within and/or extend through a sleeve of a surgical portal apparatus (not shown). Sleeve insert **300** includes substantially open proximal and distal ends **300a**, **300b** and defines a passage **303** extending therebetween. Proximal end **300a** of sleeve insert **300** includes a flange **305** extending at least partially thereabout. Flange **305** is configured for engaging a portal housing of an surgical portal apparatus upon insertion of sleeve insert **300** into the sleeve of the surgical portal apparatus. Distal end **300b** of sleeve insert **300** may include one or more tabs **307** configured to selectively engage the distal end of a sleeve (not shown), thereby, selectively securing sleeve insert **300** within the sleeve.

Sleeve insert **300** further includes a suture management system **310.** Sleeve insert **300** and suture management system **310** are substantially similar in form and function to sleeve **104** and suture management system **110,** hereinabove described. Suture management system **310** includes a plurality of longitudinal channels **312** extending the length of sleeve insert **300.** Each of channels **312** includes a slots **312a** extending at least partially along the length thereof for receiving one or more sutures "**S**" (FIG. 1).

In operation, sleeve insert **300** may be may be inserted into a sleeve of a surgical portal apparatus (not shown) prior to, during, or upon insertion of the surgical portal apparatus into a body cavity of patient (not shown). Once received within the sleeve of a surgical portal apparatus, sleeve insert **300** operates in a manner similar to sleeve **104** of surgical portal apparatus **100.** Sleeve insert **300** may be removed from the sleeve at any time during a procedure, or instead, may be disposed of with a surgical portal apparatus. Sleeve insert **300** may be disposable, or in the alternative, reusable.

Referring now to **FIG. 6****,** yet another embodiment of the present disclosure is shown generally as sleeve insert **400.** Sleeve insert **400** is substantially similar to sleeve insert **300,** and therefore, will only be described as relates to the differences therebetween. Sleeve insert **400** includes a pair of tabs **405** on a proximal end thereof for engaging a portal housing or the proximal end of a sleeve of a surgical portal apparatus (not shown). Sleeve insert **400** includes a suture management system **410.** Suture management system **410** includes a plurality of longitudinal channels **412.** Each of channels **412** includes a slot **412a** extending at least partially along a length thereof.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

## Claims

1. A surgical portal apparatus for use in surgical procedures incorporating at least one suture, which comprises:
a portal housing defining a longitudinal axis and having a longitudinal opening therethrough for receiving a surgical object; and
a sleeve extending from the portal housing and including one or more channels formed along the length of the sleeve, the channels being configured to receive the at least one suture.

2. The surgical portal apparatus of claim 1, wherein the one or more channels includes a slot for receiving the at least one suture.

3. The surgical portal apparatus of claim 1 or claim 2, wherein the one or more channels include open proximal and distal ends.

4. The surgical portal apparatus of any preceding claim, wherein the one or more channels are integrally formed with the sleeve.

5. The surgical portal apparatus of any of claims 1 to 3, wherein the one or more channels are fixedly secured to the sleeve.

6. The surgical portal apparatus of any preceding claim, wherein the one or more channels includes a circular cross-sectional profile.

7. A suture retaining insert for use in a surgical portal apparatus, comprising:
a sleeve configured to be selectively received in a longitudinal opening of a surgical portal apparatus, the sleeve including a longitudinal passage; and
one or more channels integrally formed along the length of the sleeve and configured to receive at least one suture.

8. The suture retaining insert of claim 7, wherein the one or more channels include a slot for receiving the at least one suture therein.

9. The suture retaining insert of claim 8, wherein the one or more slots are formed along an exterior of the sleeve.

10. The suture retaining insert of claim 8, wherein the one or more slots are formed along the longitudinal passage.

11. The suture retaining insert of any of claims 7 to 10, further including one or more tabs configured for operable engagement with the portal housing of the surgical portal apparatus.
